# EUROPEAN PATENT APPLICATION

(11) **EP 4 846 982 A1**
(43) Date of publication of application: **02.12.2026**
(21) Application number: 26181463.6
(22) Date of filing: 27.05.2026
(51) Int. Cl.: A61F 2/54, A61F 5/01

(54) **ORTHOSIS PROSTHESIS FOR COMPENSATING FOR IMPAIRED FUNCTIONALITY OF A HUMAN UPPER LIMB**

(30) Priority: 30.05.2025 DE 102025121310
(71) Applicant: macu4 AG, 8001 Zürich (CH)
(72) Inventor: Chevrot, Alec, 2505 Biel (CH); Schiller, Lukas, 6300 Zug (CH)
(74) Representative: Kreuels, Justus

(57) **Abstract**

Orthosis prosthesis (1) for compensating for impaired functionality of a human upper limb, having a lower shell (3) which is configured to receive a distal limb portion (2) and an upper shell (6) which is configured to at least partially surround a proximal limb portion (7) of the human individual, wherein the lower shell and the upper shell are connected to one another by at least one connecting element (8), wherein the at least one connecting element (8) is connected to the lower shell and to the upper shell in each case at connection points (9) with joints (10), wherein at least one connection point (9) is embodied with a ball joint which allows at least two rotational degrees of freedom between the lower shell or the upper shell and the at least one connecting element.

## Description

The invention relates to an Orthosis prosthesis for compensating for a impaired functionality of a human upper limb. The invention relates in particular to an Orthosis prosthesis for replacing a part of a forearm. The Orthosis prosthesis described here can be used, for example, to replace a part of a forearm after an amputation and/or after a partial amputation of a hand.

In principle, it is desirable for such an Orthosis prosthesis to be adapted as individually as possible to the patient. This is of course associated with a considerable outlay. Therefore, modular systems in which partially prefabricated (non-patient-individual) separate parts can be joined together to form the Orthosis prosthesis are routinely advantageous. An Orthosis prosthesis should offer the best possible wearing comfort for the patient. At the same time, the range of applications of the Orthosis prosthesis should be as wide as possible for different tasks and activities.

It is an object of the present invention to propose an Orthosis prosthesis and a method for producing an Orthosis prosthesis which satisfies the requirements described as well as possible.

This object is achieved by the invention according to the features of the independent patent claims. Further advantageous embodiments are specified in the dependent patent claims and in the description and in particular also in the description of the figures. It should be pointed out that a person skilled in the art combines the individual features with one another in a technologically meaningful manner and thus arrives at further embodiments of the invention.

Described here is an Orthosis prosthesis for compensating for impaired functionality of a human upper limb, for example for replacing an amputated part of a forearm, having a lower shell which is configured to receive a distal limb portion and an upper shell which is configured to at least partially surround a proximal limb portion of the human individual, wherein the lower shell and the upper shell are connected to one another by at least one connecting element, wherein the at least one connecting element is connected to the lower shell and to the upper shell in each case at connection points with joints, wherein at least one connection point is embodied with a ball joint which allows at least two rotational degrees of freedom between the lower shell or the upper shell and the at least one connecting element.

Described here is, in particular, an Orthosis prosthesis for replacing a part of the forearm of a patient, in particular for replacing an amputated part of a forearm, having a forearm shell which is configured to receive a stump of the forearm and on which further Orthosis prosthesis elements are arranged or can be arranged, and an upper arm shell which is configured to at least partially surround the upper arm of the patient, wherein the forearm shell and the upper arm shell are connected to one another by at least one connecting element, wherein the at least one connecting element is connected to the forearm shell and to the upper arm shell in each case at connection points with joints, wherein at least one connection point is embodied with a ball joint which allows at least two rotational degrees of freedom between the forearm shell or the upper arm shell and the at least one connecting element.

In order to describe the invention, in the text which follows, in part the forearm is used as an example of a distal limb portion and the upper arm is used as an example of a proximal limb portion, and the described Orthosis prosthesis is explained using the example of the elbow joint. In principle, however, the described Orthosis prosthesis is suitable for various joints on the upper limbs/upper extremities of the human individual as an orthosis or as a prosthesis, for example for the wrist, with the hand then being the distal limb portion and the forearm being the proximal limb portion. The application is also possible on the shoulder joint, finger joints, etc.

Preferably, a plurality of, or in particular even all, connection points between connecting elements and the lower shell and the upper shell are embodied with ball joints which allow at least two rotational degrees of freedom between the lower shell or the upper shell and the at least one connecting element.

Preferably, the ball joints, as two rotational degrees of freedom, allow, in particular, a rotation about a first ball axis (also referred to as a pitching movement or flexion-extension) and a rotation about a second axis (also referred to as lateral inclination or abduction-adduction).

It is further preferred if at least one ball joint is configured at a connection point such that it allows three rotational degrees of freedom between the lower shell or the upper shell and the at least one connecting element.

Preferably, a plurality of, or in particular even all, connection points between connecting elements and the lower shell and the upper shell are embodied with ball joints which allow three rotational degrees of freedom between the lower shell or the upper shell and the at least one connecting element.

The third (preferably likewise present) rotational degree of freedom of the ball joint or the ball joints is the relative rotation of the components connected by the ball joints with respect to one another about themselves (also referred to as rotational movement or inner-outer rotation).

The angular ranges in which the respective rotations between the connecting elements and the upper shell or the lower shell on the ball joints are possible are usually limited on account of the construction of the ball joints. Preferably, the angular range is 360° at least for one direction of rotation (in particular for the pitching movement/tilting movement or else flexion-extension). Particularly preferably, the angular ranges in which the respective rotation is possible also depend on one another. If the ball joint has a bent position in relation to the pitching movement, it is, for example, possible that the angular range in which a rotational movement or inner-outer rotation is still possible is smaller than if the ball joint is extended.

Limitations of the angular ranges result, for example, from the fact that a spherical body of the ball joint is connected at a position to a carrier body which limits the angular ranges or hinders rotational movements in certain ranges.

The ball joints are preferably designed such that the freedom of movement of the upper shell and the lower shell with respect to one another is impaired as little as possible or the natural freedoms of movement of the joint between the distal limb portion and the proximal limb portion are preferably hindered as little as possible by the Orthosis prosthesis and the ball joints. Owing to the fact that a total of two ball joints exist between the upper shell and the lower shell in each case (in the case of both connecting elements), the mobility of the upper shell and the lower shell relative to one another is very high. By means of the connecting elements, it is possible to exert substantially a tensile force between the upper shell and the lower shell. By means of this tensile force, the holding force with which the lower shell is seated on the distal limb portion (for example on the stump of a forearm) can be greatly improved. At the same time, a blockage or restriction of the mobility of the joint (for example of the elbow joint) is small or minimal.

The upper shell and the lower shell are preferably made of a flexible material. If appropriate, flexible inserts (possibly adapted to the individual patient) may also be arranged in the upper shell and the lower shell, said inserts improving contact with the distal limb portion (for example on a stump of the forearm) and the proximal limb portion.

The Orthosis prosthesis described here makes it possible, in particular, to provide efficient orthopedic care for pediatric patients and also very small children. Costs and delivery times can be reduced. The upper shell increases the stability of the Orthosis prosthesis especially in small children with a short stump of the forearm as the lower limb portion on which the lower shell is arranged.

It is particularly preferable if the upper shell and the lower shell are connected to one another by two connecting elements, wherein the connection points by means of which the two connecting elements are connected to the lower shell define a first axis intersecting a distal limb portion received in the lower shell, and wherein the connection points by means of which the two connecting elements are connected to the upper shell define a second axis intersecting a proximal limb portion received in the upper shell.

It is further preferred if a flexion axis of a joint of the patient between the distal limb portion and the proximal limb portion is arranged in a region between the first axis and the second axis when the distal limb portion and a proximal limb portion of the patient are received in the lower shell and in the upper shell.

The first axis and the second axis are each imaginary axes defined by the connection points. In certain positions of the lower shell and the upper shell relative to one another, the two axes are parallel to one another. In other positions, an angle of the two axes to one another arises, so that the two axes are skewed. However, the connecting elements always produce a distance between the two axes, so that the two axes do not intersect. Preferably, a joint center of the joint, through which a main flexion axis of the joint runs, lies between the two axes or in a space between the two axes when the Orthosis prosthesis is placed on an arm. Preferably, the main flexion axis and the joint center lie as centrally as possible between the two axes and also as centrally as possible between the total of four connection points with which the two connecting elements are connected to the upper shell and the lower shell. Thus, particularly high and natural kinematics between the upper shell and the lower shell can be achieved.

In principle, this construction of a connection between shells with connecting elements and ball joints for orthoprostheses or orthoses can be used on many joints of the human body. Particularly for the orthoprostheses described here, however, this construction of a joint has the considerable advantages already described further above.

It is particularly preferred if the two connecting elements are arranged in such a way that they are arranged approximately parallel during flexion of the elbow joint.

If an "approximately parallel" arrangement is mentioned here, angular deviations of, for example, up to 10 degrees are also included here and they are also considered as "parallel" if appropriate. It is particularly important that the angle between the two connecting elements does not change during flexion of the elbow element, but rather the two connecting elements are moved more or less parallel to one another.

It is further preferred if the at least one connecting element and the joints are embodied such that a relative rotation between a first main axis of the lower shell and a second main axis of the upper shell is allowed in order to allow pronation and supination of a distal limb portion (for example a stump of a forearm) arranged in the lower shell.

As a result of pronation or supination, a skewed position of the first axis and of the second axis relative to one another preferably occurs. At the same time, a skewed position of the connecting elements relative to one another preferably also arises. The interaction of a total of four connection points between the upper shell and the lower shell, via the connecting elements and the possibility of permitting a skewed position of the first axis and the second axis with respect to one another or of the connecting elements with respect to one another, also reduces or avoids a possible hindrance/impairment of the pronation and the supination of the forearm or of the distal limb portion by means of the Orthosis prosthesis.

It has already been described further above that it is preferred if the at least one connecting element is configured to transmit a tensile force from the upper shell to the lower shell, wherein this tensile force supports a holding force with which the lower shell is held on the distal limb portion (for example on the stump of the forearm).

It is further preferred if the upper shell has a cuff body and a closure, wherein the cuff body can be placed around the upper arm and can be closed with the closure in order to fix the upper arm in the upper shell.

Preferably, the upper shell can be fixed to the upper arm in such a way that a tensile force is transmitted from the upper shell to the upper arm. Preferably, a clamping force with which the upper arm is clamped by the upper shell can be set. This can be done with the closure. By designing the upper shell with a cuff body and a closure, the application of the upper shell can be simplified. Preferably, the upper shell can be applied to the upper arm when the lower shell has already been applied to the distal limb portion. It is preferably possible to use the Orthosis prosthesis only with the lower shell in order then to additionally apply the upper shell if necessary (if an increased holding force is required).

It is also preferred if at least one ball joint is embodied at a connection point such that a connection between the connecting element and the lower shell and/or the upper shell can be established and disconnected without tools.

Preferably, the connecting elements can be attached if the upper shell and the lower shell are both applied to the distal limb portion (for example to the stump of the forearm) and to the proximal limb portion. This can take place in particular during the first application of the Orthosis prosthesis to the arm of a patient. Preferably, a connected state of the upper shell and of the lower shell remains established during regular use of the Orthosis prosthesis via the connecting elements when the Orthosis prosthesis is applied and taken off.

It is further preferable if at least one ball joint is embodied with a spherical body on the lower shell or on the upper shell and with a hollow sphere inner surface, corresponding to the spherical body, on the connecting element, wherein the hollow sphere inner surface is formed partially by a main body of the connecting element and partially by a closing body, wherein the closing body can be removed from the main body in order to allow tool-free assembly and disassembly of the ball joint.

The closing body is preferably held with a clamping force in receptacles on the main body of the connecting element. The receptacles preferably represent a free space in which both the spherical body and the closing body are inserted. When the closing body is removed from the receptacle, the spherical body can preferably be removed from the connecting element or from the receptacle. When the closing body is inserted into the receptacle, the spherical body is held in the receptacle or in the hollow sphere inner surface formed partially by the main body and partially by the closing body.

It is further preferable if at least one ball joint is embodied with a spherical body on the lower shell or on the upper shell and with a hollow sphere inner surface, corresponding to the spherical body, on the connecting element, wherein the spherical body is fastened to a carrier body formed on the lower shell or on the upper shell and can be exchanged, wherein differently designed spherical bodies can be fastened to the carrier body in order to achieve different properties of the ball joint.

The connecting element (and thus also the hollow sphere inner surface) and/or the spherical body are preferably produced from flexible materials which are under (slight) stress in the assembled state of the ball joint. As a result, a certain friction is preferably generated in the ball joint and results in (low) forces being required in order to move the lower shell and the upper shell relative to one another. Preferably, forces assist the wearer of the Orthosis prosthesis in holding the arm or the elbow joint in a specific position.

It is preferably possible for spherical bodies of the ball joint to be selected in a targeted manner in order to achieve specific friction and possibly latching positions of the ball joints during certain movements. Preferably, the choice of suitable spherical bodies also allows partial (or, if appropriate, complete) stiffening between the upper shell and the lower shell to be achieved.

In embodiment variants, a clamping screw can be arranged on the spherical body, by means of which clamping screw the spherical body can be placed under tension in order to deform the spherical body and thus possibly increase the friction in the ball joint in a targeted manner. In embodiment variants, it is possible rather to use cylindrically shaped spherical bodies and/or spherical bodies with a polygonal shape in order to increase the friction or possibly to provide latching positions of the ball joints in certain positions.

It is further preferred if the at least one connecting element is deformable in order to adapt a distance, provided by the connecting element, between the connection points on the lower shell and the upper shell. If appropriate, connecting elements between the connection points may have a flexible (if appropriate elastic) portion and/or be produced completely from a flexible/elastic material.

If appropriate, connecting elements with an adjustable length can also be used. It is further preferred if the connecting elements allow a defined torsion or bending and possibly have damping properties. Connecting elements with different lengths which ensure different distances between the connection points can be used on an Orthosis prosthesis.

The intention here is also to describe a method for producing a described Orthosis prosthesis for a patient, having the following steps:
a) measuring a distal limb portion (for example a stump of a forearm) and a proximal limb portion of the patient;
b) selecting a matching lower shell from a set of different lower shells in accordance with the measurement carried out in step a);
c) selecting a matching upper shell from a set of different upper shells in accordance with the measurement carried out in step a);
d) placing the lower shell and the upper shell on the arm of the patient;
e) testing different connecting elements for connecting the lower shell and the upper shell to the arm of the patient; and
f) assembling suitable connecting elements with the selected lower shell and the selected upper shell to form the Orthosis prosthesis.

The advantages and design features disclosed in connection with the described Orthosis prosthesis can be applied and transferred to this method.

The sets of lower shells and upper shells described in steps b) and c) relate in particular to different standard shells from which a particularly suitable shell can be selected in order to form an Orthosis prosthesis adapted to the individual patient. For example, n different standard shells for the upper shell and m different standard shells for the lower shell can be available. It is described here as an embodiment variant that standard shells are used. Of course, it is also possible to use an individually manufactured/personalized shell for a patient for at least one of the two shells (upper shell and lower shell).

The testing of the different connecting elements in step e) comprises in particular that the patient wearing the Orthosis prosthesis carries out certain test movements and test activities with the Orthosis prosthesis and in the process it is checked how well these test movements/test activities are possible with the respective connecting elements between the upper shell and the lower shell.

The invention and the technical field of the invention are explained in more detail below with reference to the figures. The figures show preferred exemplary embodiments, to which the invention is not impaired. It should be noted in particular that the figures and in particular the size ratios illustrated in the figures are only schematic. In the drawings:
- Fig. 1: shows a first view of a described Orthosis prosthesis on an upper limb of a patient;
- Fig. 2: shows a second view of a described Orthosis prosthesis on an upper limb of a patient;
- Fig. 3: shows a connecting element on a described Orthosis prosthesis in detail;
- Fig. 4: shows a ball joint on a connecting element of a described Orthosis prosthesis in cross section; and
- Figs. 5a to 5c: show embodiment variants of a spherical body of a ball joint for a described Orthosis prosthesis.

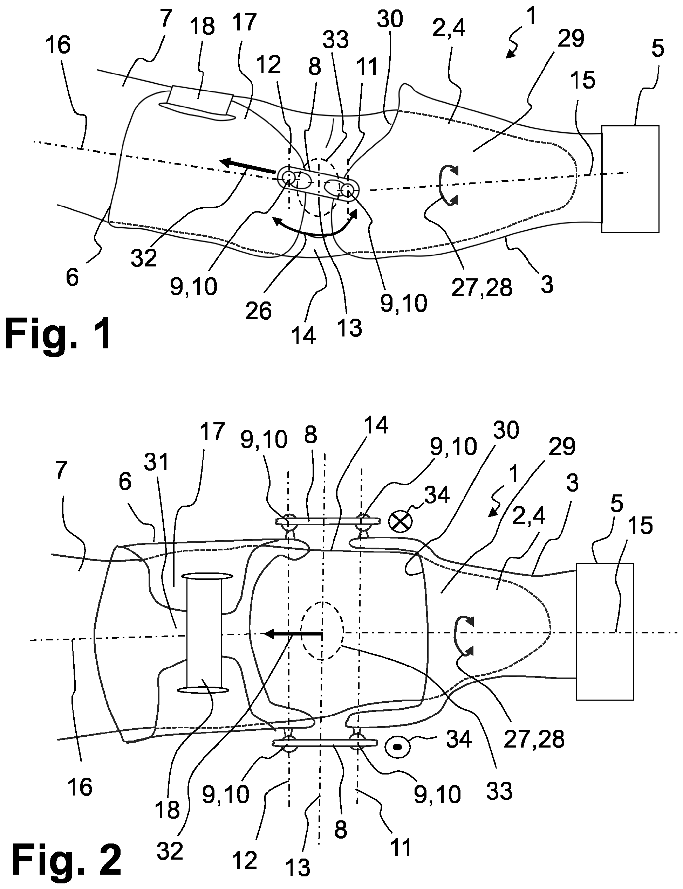

Fig. 1 and Fig. 2 show a described Orthosis prosthesis 1 on the upper limb (on an arm) of a patient in a first view and in a second view. By way of example, such an Orthosis prosthesis 1 is shown here on an elbow of a human individual, with the lower shell 2 being a forearm shell for receiving a forearm 2 and the upper shell 6 being an upper arm shell 6 for receiving the upper arm 7. However, the invention is not restricted to this application, but can in principle be used for all joints on an upper limb/extremity in which the distal limb portion 2 and the proximal limb portion 7 can be enclosed in a supported manner with such an Orthosis prosthesis in order to compensate for a impaired functionality of an upper limb of a person. The arm of the patient consisting of the upper arm 7 and a stump 4 of the forearm 2 is schematically indicated here. The stump 4 of the forearm 2 lies here in a forearm shell 3. The upper arm 7 is surrounded by an upper arm shell 6. In the following, the forearm 2 represents, by way of example, the distal limb portion. In the following, the upper arm 7 represents, by way of example, the proximal limb portion. In the following, the forearm shell 3 represents, by way of example, the lower shell. In the following, the upper arm shell 6 represents, by way of example, the upper shell.

Further prosthetic elements 5 can be fastened to the forearm shell 3. This further prosthetic element 5 can be, for example, an artificial hand or a tool. The forearm shell 3 preferably has a cavity 29 with a (proximal) opening 30 oriented toward the body of the patient or toward the upper arm 7. The cavity 29 is preferably adapted to the shape of the stump 4 of the forearm 2. The forearm 2 or the stump 4 can be introduced through the opening 30 into the cavity 29 or into the forearm shell 3. Preferably, the forearm shell 3 then receives the forearm 2 or the stump 4 in the cavity 29 in a form-fitting manner.

The upper arm shell 6 is embodied here as a cuff 17 which can be placed around the upper arm 7, with this in particular also being possible without pushing the upper arm shell 6 over the forearm 2, in particular when the forearm shell 3 has already been applied to the forearm 2 or to the stump 4. For this purpose, the cuff 17 can be bent apart such that a lateral access 31 of the cuff 17 of the upper arm shell 6 is opened. The cuff 17 can then be bent together again such that the lateral access 31 becomes smaller again or is closed. Preferably, the upper arm shell 6 has a closure 18 (formed for example as a buckle) by means of which the upper arm shell 6 or the cuff 17 can then be secured to the upper arm 7.

The forearm shell 3 and the upper arm shell 6 are connected to one another by two connecting elements 8. The connecting elements 8 each have two connection points 9 to the forearm shell 3 and the upper arm shell 6, respectively. Ball joints 10, which each allow two (preferably three) rotational degrees of freedom of movement, are arranged on each of the connection points 9. A first (imaginary) axis 11 runs through the two connection points 9 by means of which the two connecting elements 8 are connected to the forearm shell 3. A second (imaginary) axis 12 runs through the two connection points 9 by means of which the two connecting elements 8 are connected to the upper arm shell 6. The first axis 11 intersects the forearm 2 inserted into the forearm shell 3. The second axis 12 intersects the upper arm 7 inserted into the upper arm shell 6. An elbow joint 14 of the patient lies spatially between the first axis 11 and the second axis 12. A flexion axis 13 of the elbow joint 14 is regularly not quite clearly defined and is displaced during flexion 26 of the elbow. By virtue of the two axes 11, 12 with the ball joints 10, there are many possibilities of movement of the upper shell 6 and the lower shell 3 relative to one another. The connecting element 8 substantially applies a tensile force 32 from the upper shell 6 to the lower shell 3, which tensile force assists the lower shell 3 in being held securely on the stump 4 of the forearm 2. As a result of the fact that there are two connecting elements 8 which preferably both partially apply the tensile force 32, it is preferably ensured that the resulting tensile force preferably acts centrally, approximately on a joint center 33 of the elbow joint 14.

The connection of the upper arm shell 6 and the forearm shell 3 to the connecting elements 8 and the ball joints 10 at the connection points 9 preferably also allows, in addition to allowing flexion 26 about the flexion axis 13, a rotation of the forearm 2 or of the forearm shell 3 about a first main axis 15 of the forearm 2 or of the forearm shell 3. In other words: The upper arm 7 or the upper arm shell 6 preferably has a second main axis 16. The forearm 2 or the forearm shell 3 preferably has a first main axis 15. A rotation of these two main axes 15, 16 relative to one another is possible. Figs. 1 and 2 preferably show a stretched (non-bent) state of the arm. By virtue of the connecting elements 8 and the ball joints 10 at the connection points 9, the lower shell 3 can be rotated relative to the upper shell 6 and allow pronation 27 and supination 28 (rotation of the forearm 2). In this case, a relative angle between the two connecting elements 8 is changed. Directions of movement 34 of the connection points 9 of the connecting elements 8 on the forearm shell 3 are indicated schematically by arrows in Fig. 2. In the case of pronation 27 or supination 28, here one connection point 9 is moved upward and the other connection point 9 is moved downward.

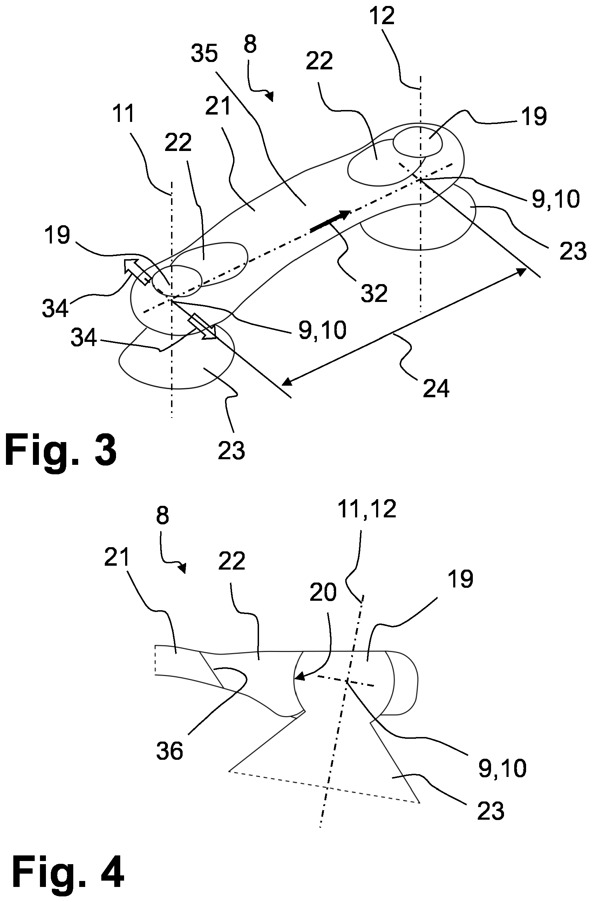

Fig. 3 shows a described connecting element 8 with its connection points 9 to the upper arm shell 6 (not illustrated here) and to the forearm shell 3 (not illustrated here). At each of the connection points 9 there are formed ball joints 10 which connect the connecting element 8 to the upper arm shell 6 or the forearm shell 3 via carrier bodies 23.

The first axis 11 defined by the two connection points 9 (both connecting elements 8) on the forearm shell 3 and the second axis 12 defined by the two connection points 9 (both connecting elements 8) on the upper arm shell 6 are illustrated schematically here. The directions of movement 34 of connection points 9 indicated in Fig. 2 during pronation/supination of the forearm are likewise schematically illustrated here for orientation. The tensile force 32 exerted by the connecting elements 8 on the forearm shell 3 is likewise schematically illustrated here.

The carrier bodies 23 are domes which are seated on the upper arm shell 6 and the forearm shell 3 and on which a spherical body 19 is seated in each case.

In embodiment variants, the spherical body 19 can also be attached directly to the upper shell 6/upper arm shell 6 or to the lower shell 3/forearm shell 3, without separate carrier bodies 23 being required. The carrier bodies 23 and/or possibly also the spherical bodies 19 can also be integrally formed on the shells 3, 6.

This spherical body 19 is surrounded by a hollow sphere inner surface 20 formed on the connecting element 8. This is illustrated in more detail below with reference to Fig. 4. The connection points 9 or the ball joints 10 are at a distance 24 from one another. This distance 24 substantially defines the kinematics of the movement of the forearm shell 3 and the upper arm shell 6 relative to one another. If appropriate, the connecting elements 8 may be flexible or have a flexible (in particular) elastic portion 35. The distance 24 between the connection points 9 is thus preferably variable. This further expands the mobility, provided by the connecting elements 8 and the ball joints 10, of the forearm shell 3 and the upper arm shell 6 relative to one another.

Fig. 4 shows a cross section through the ball joint 10 at a connection point 9 between the upper arm shell 6 or the forearm shell 3 and the connecting element 8 (only partially illustrated here). The spherical body 19, which is seated on a carrier body 23, can be seen. On the connecting element 8 there is formed a hollow sphere inner surface 20 in which the spherical body 19 is seated in order to form the ball joint 10. The connecting element 8 consists substantially of a main body 21 which connects the two connection points 9 to one another and which extends from the first connection point 9 to the second connection point 9. The hollow sphere inner surfaces 20 are preferably formed only partially by the main body 21.

Preferably, the hollow sphere inner surfaces 20 are completed by closing bodies 22 which can be inserted into receptacles 36 provided for this purpose on the main body 21. The closing bodies 22 are preferably held in the receptacles 36 by a clamping force. However, the closing bodies 22 can preferably be inserted and removed without tools. When the closing bodies 22 are removed, the spherical body 19 can be removed from the connecting element 8 without tools, and the respective shell (upper arm shell 6 or forearm shell 3) can be released from the connecting element 8. When the closing body 22 is inserted into the receptacle 36, the spherical body 19 is securely held in the hollow sphere inner surface 20 of the connecting element 8.

Preferably, all connecting elements 8 and all ball joints 10 of the Orthosis prosthesis are of identical design - particularly preferably in accordance with the embodiment variant illustrated in Figs. 3 and 4.

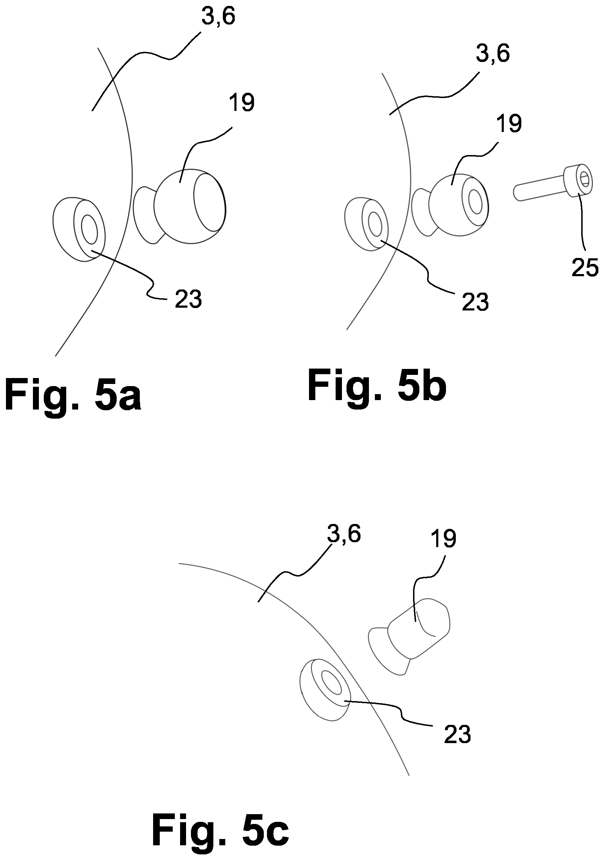

Figs. 5a to 5c show various embodiment variants of a spherical body 19 of a ball joint 10 for a described Orthosis prosthesis 1. Particularly preferably, the spherical body 19 is placed on a carrier body 23 placed with the upper arm shell 6 or the forearm shell 3. Further preferably, the spherical body 19 can be removed from the carrier body 23, as is shown in all three figures 5a to 5c.

The spherical body 19 can be fixed to the carrier body 23 by means of the screw 25 shown in Fig. 5b. If appropriate, it is possible for a tightening torque of the screw 25 to be changed in order to influence the shape of the spherical body 19. For this purpose, the spherical body 19 is preferably produced from an elastic material. Adapting the shape of the spherical body 19 makes it possible in particular to adapt internal friction in the ball joint 10 and thus to adapt the force required to move the ball joint 10 (and thus the force for moving the upper arm shell 6 and the forearm shell 3 relative to one another). Thus, the ball joint 10 can be adjusted individually to the patient.

Fig. 5c shows a variant in which a spherical body 19 whose shape is adapted is placed on the carrier body 23. The spherical body 19 illustrated in Fig. 5 is not primarily spherical, but rather has the shape of a cylinder. By virtue of such a spherical body 19, the rotational degrees of freedom of the ball joint 10 can be restricted or it can be ensured that the friction in the ball joint 10 differs depending on the direction of movement. Thus, a further adaptation of the Orthosis prosthesis that is individual to the patient is possible.

The Orthosis prosthesis described here is fundamentally distinguished by a particularly good adaptability to the individual patient. At the same time, very good wearing comfort and extensive usability for a wide variety of applications are achieved.

### List of reference signs

- 1: Orthosis prosthesis
- 2: Distal limb portion/forearm
- 3: Lower shell/forearm shell
- 4: Stump
- 5: Prosthetic element
- 6: Upper shell/upper arm shell
- 7: Proximal limb portion/upper arm
- 8: Connecting element
- 9: Connection point
- 10: Ball joint
- 11: First axis
- 12: Second axis
- 13: Flexion axis
- 14: Elbow joint
- 15: First main axis
- 16: Second main axis
- 17: Cuff
- 18: Closure
- 19: Spherical body
- 20: Hollow sphere inner surface
- 21: Main body
- 22: Closing body
- 23: Carrier body
- 24: Distance
- 25: Screw
- 26: Flexion
- 27: Pronation
- 28: Supination
- 29: Cavity
- 30: Opening
- 31: Access
- 32: Tensile force
- 33: Joint center
- 34: Direction of movement
- 35: Flexible portion
- 36: Receptacles

## Claims

1. An Orthosis prosthesis (1) for compensating for impaired functionality of a human upper limb, having a lower shell (3) which is configured to receive a distal limb portion (2) and an upper shell (6) which is configured to at least partially surround a proximal limb portion (7) of the human individual, wherein the lower shell (3) and the upper shell (6) are connected to one another by at least one connecting element (8), wherein the at least one connecting element (8) is connected to the lower shell (3) and to the upper shell (6) in each case at connection points (9) with joints (10), wherein at least one connection point (9) is embodied with a ball joint (10) which allows at least two rotational degrees of freedom between the lower shell (3) or the upper shell (6) and the at least one connecting element (8).

2. The Orthosis prosthesis (1) as claimed in claim 1, wherein the upper shell (6) is an upper arm shell for receiving an upper arm (7) and the lower shell (3) is a forearm shell for receiving a forearm (2), in particular for receiving a stump (4) of a forearm (2).

3. The Orthosis prosthesis (1) as claimed in either of the preceding claims, wherein all connection points (9) between connecting elements (8) and the lower shell (3) and the upper shell (6) are embodied with ball joints (10) which allow at least two rotational degrees of freedom between the upper shell (3) or the upper limb portion (6) and the at least one connecting element (8).

4. The Orthosis prosthesis (1) as claimed in one of the preceding claims, wherein at least one ball joint (10) is configured at a connection point (9) such that it allows three rotational degrees of freedom between the lower shell (3) or the upper shell (6) and the at least one connecting element (8).

5. The Orthosis prosthesis (1) as claimed in one of the preceding claims, wherein the upper shell (3) and the lower shell (6) are connected to one another by two connecting elements (8), wherein the connection points (9) by means of which the two connecting elements (8) are connected to the lower shell (3) define a first axis (11) intersecting a distal limb portion (2) received in the lower shell (3), and wherein the connection points (9) by means of which the two connecting elements (8) are connected to the upper shell (6) define a second axis (12) intersecting a proximal limb portion (7) received in the upper shell (6).

6. The Orthosis prosthesis (1) as claimed in claim 5, wherein a flexion axis (13) of a joint (14) of the patient between the distal limb portion (2) and the proximal limb portion (7) is arranged in a region between the first axis (11) and the second axis (12) when a distal limb portion (2) and a proximal limb portion (7) of the patient are received in the lower shell (3) and in the upper shell (6).

7. The Orthosis prosthesis (1) as claimed in claim 6, wherein the two connecting elements (8) are arranged in such a way that they are arranged approximately parallel during flexion (26) of the joint (14).

8. The Orthosis prosthesis (1) as claimed in one of the preceding claims, wherein the at least one connecting element (8) and the joints (10) are embodied such that a relative rotation between a first main axis (14) of the lower shell (3) and a second main axis (15) of the upper shell (6) is allowed so as to allow pronation (27) and supination (28) of a distal limb portion (2) in the lower shell (3).

9. The Orthosis prosthesis (1) as claimed in one of the preceding claims, wherein the at least one connecting element (8) is configured to transmit a tensile force from the upper shell (6) to the lower shell (3), wherein this tensile force supports a holding force with which the lower shell (3) is held on the distal limb portion (2).

10. The Orthosis prosthesis (1) as claimed in one of the preceding claims, wherein the upper shell (3) has a cuff body (17) and a closure (18), wherein the cuff body (17) can be placed around the proximal limb portion (7) and can be closed by the closure (18) in order to fix the proximal limb portion (7) in the upper shell (6).

11. The Orthosis prosthesis (1) as claimed in one of the preceding claims, wherein at least one ball joint (10) is embodied at a connection point (9) in such a way that a connection between the connecting element (8) and the upper shell (3) and/or the lower shell (6) can be established and disconnected without tools.

12. The Orthosis prosthesis (1) as claimed in claim 11, wherein at least one ball joint (10) is embodied with a spherical body (19) on the lower shell (3) or on the upper shell (6) and with a hollow sphere inner surface (20), corresponding to the spherical body (19), on the connecting element (8), wherein the hollow sphere inner surface (20) is formed partially by a main body (21) of the connecting element (8) and partially by a closing body (22), wherein the closing body (22) can be removed from the main body (21) in order to allow tool-free assembly and disassembly of the ball joint (10).

13. The Orthosis prosthesis (1) as claimed in either of claims 11 and 12, wherein at least one ball joint (10) is embodied with a spherical body (19) on the lower shell (3) or on the upper shell (6) and with a hollow sphere inner surface (20), corresponding to the spherical body (19), on the connecting element (8), wherein the spherical body (19) is fastened to a carrier body (23) formed on the forearm shell (3) or on the upper arm shell (6) and can be exchanged, wherein differently designed spherical bodies (19) can be fastened to the carrier body (23) in order to achieve different properties of the ball joint (10).

14. The Orthosis prosthesis (1) as claimed in one of the preceding claims, wherein the at least one connecting element (8) is deformable in order to adapt a distance (24), provided by the connecting element (8), between the connection points (9) on the lower shell (3) and the upper shell (6).

15. A method for producing an Orthosis prosthesis (1) as claimed in one of claims 1 to 14 for a patient, having the following steps:
a) measuring a distal limb portion (2), in particular a stump (4) of the forearm (2), and a proximal limb portion (7) of the patient;
b) selecting a matching lower shell (3) from a set of different lower shells (3) in accordance with the measurement carried out in step a);
c) selecting a matching upper shell (6) from a set of different upper shells (6) in accordance with the measurement carried out in step a);
d) placing the lower shell (3) and the upper shell (6) on the arm of the patient;
e) testing different connecting elements (8) for connecting the lower shells (3) and the upper shell (6) to an upper limb of the patient; and
f) assembling suitable connecting elements (8) with the selected lower shell (3) and the selected upper shell (6) to form the Orthosis prosthesis (1).
